(19) Europäisches Patentamt
European Patent Office
Office européen des brevets

(11) Publication number: **0 339 973**
**A1**

(12) **EUROPEAN PATENT APPLICATION**

(21) Application number: **89304163.2**

(22) Date of filing: **26.04.89**

(51) Int. Cl.4: **C 07 D 471/04**
C 07 D 519/00
//(C07D471/04,221:00,221:00),
(C07D519/00,471:00,471:00)

(30) Priority: **28.04.88 US 188579**

(43) Date of publication of application:
**02.11.89 Bulletin 89/44**

(84) Designated Contracting States:
**AT BE CH DE FR GB IT LI LU NL SE**

(71) Applicant: **EASTMAN KODAK COMPANY (a New Jersey corporation)**
**343 State Street**
**Rochester New York 14650 (US)**

(72) Inventor: **Delton, Mary Helen c/o EASTMAN KODAK COMPANY**
**Patent Department 343 State Street**
**Rochester New York 14650 (US)**

**Warren, Harold Chester III c/o EASTMAN KODAK COMPANY Patent Department 343 State Street**
**Rochester New York 14650 (US)**

(74) Representative: **Nunney, Ronald Frederick Adolphe et al**
**Kodak Limited Patent Department Headstone Drive**
**Harrow Middlesex HA1 4TY (GB)**

(54) **Substituted 1,10-phenanthrolines.**

(57) Lipophilic 1,10-phenanthrolines having one or more substituents are described. These compounds have the structure:

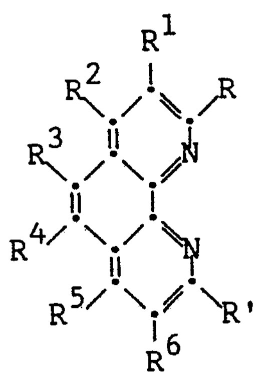

wherein
R is selected from the group consisting of alkyl having a molecular weight of at least 45, cycloalkyl having 5 to 10 carbon atoms in the ring and substituted phenyl,
R′ is either hydrogen, halo or independently selected from the groups defined for R,
$R^1$, $R^2$, $R^3$, $R^4$, $R^5$ and $R^6$ are independently hydrogen, alkyl of 1 to 10 carbon atoms, cycloalkyl of 6 to 10 carbon atoms, aryl of 6 to 14 carbon atoms or halo groups,
provided that R′ is not hydrogen when R is alkyl as defined above, and further provided that when R and R′ are both either n-butyl, t-butyl or alkyl-substituted phenyl, at least one of $R^1$, $R^2$, $R^3$, $R^4$, $R^5$ and $R^6$ is alkyl, cycloalkyl, aryl or halo as defined above.

EP 0 339 973 A1

**Description**

**SUBSTITUTED 1,10-PHENANTHROLINES**

This invention relates to novel lipophilic compounds. More particularly, these compounds are 1,10-phenanthrolines which have one or more lipophilic substitutents. These compounds are useful in a number of ways, including their use for complexing various metals, and as ionophores in lithium ion-selective electrodes.

1,10-Phenanthrolines are well known aromatic heterocycles which have been used in various medical and analytical procedures. See, for example, the Encyclopedia of Industrial Chemical Analysis, Snell & Ettre (Eds.) Interscience Publishers, New York, 1973 (Vols. 4, p. 39, 9, p. 274, 10, p. 201 and 18, pp. 408 & 427) for analytical procedures for detecting various ions using 1,10-phenanthrolines.

Certain substituted 1,10-phenanthrolines are also described in the art. For example, Alessio et al [J.Mol.Catalysis, 29, pp. 77-98 (1985)] describe the use of transition metals with 2,9-dimethyl-, 4,7-dimethyl- and 3,4,5,6,7,8-hexamethyl-1,10-phenanthrolines to form a catalyst system for the reduction of nitrobenzene to aniline. The oxidation-reduction mechanisms of metal-1,10-phenanthroline derivatives (4,7-phenyl-, 4-methyl- and 2-methyl-) are described by Rollick et al [J.Am.Chem.Soc., 104, pp. 1319-1330 (1982)].

Dietrich-Buchecker et al [Tetra. Lett., 23(50), pp. 5291-5294, (1982)] also describe the preparation of 2,9-diphenyl-, 2,9-di-n-butyl-, 2,9-di-t-butyl- and 2,4,7,9-tetraphenyl-1,10-phenantrolines. Considerable literature exists showing these or similar compounds and their use in catalytic or metal complexation procedures.

Some phenanthrolines have been described for use in metal ion electrodes. For example, Pfeiffer et al (Zeitschrift für anorganische und allgmeine Chemie, 239, pp. 133-144, 1938) describe the preparation of lithium complexes with a perchlorate of o-phenanthroline. Also, an abstract of U.S.S.R. Patent 1,124,214 (published November 15, 1984) describes a magnesium ion-selective membrane composition comprising a complex of magnesium and a phenathroline modified with a tetraphenyl borate (as the membrane active compound) and a carrier solvent. The magnesium-phenathroline complex appears to be the matrix for the ion-selective borate compound. An abstract of U.S.S.R. 989,441 (published January 15, 1983) describes the use of bis(2,9-dimethyl-1,10-phenanthrolin)cupripicrate in a copper(I) ion-selective membrane and electrode.

U.S. Patent 3,483,112 relates to an anion-selective electrode sensitive to perchlorate, halide, nitrate and other anions. The selectivity is allegedly obtained using an ion exchanger liquid comprising a salt of the anion and a metal (such as copper, ion or cobalt) complexed with an oleophilic substituted phenanthroline.

Sugihara et al, Chem. Letters, 1987, pp. 2391-2392 describes the use of 2,9-dimethyl- and 2,9-di-n-butyl-1,10-phenanthrolines as ionophores in lithium ion selective compositions and electrodes.

There is a continued need in the art, however, for novel phenanthroline compounds which provide advantages in ion selectivity, metal complexation or other uses, and which provide greater versatility in selecting ionophores for lithium ion selective compositions and electrodes.

In this invention, novel substituted 1,10-phenanthrolines are characterized by having the structure:

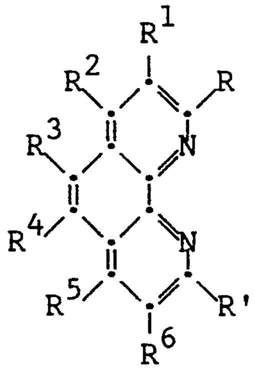

wherein

R is selected from the group consisting of alkyl having a molecular weight of at least 45, cycloalkyl having 5 to 10 carbon atoms in the ring and substituted phenyl,

R′ is either hydrogen, halo or independently selected from the groups defined for R,

$R^1$, $R^2$, $R^3$, $R^4$, $R^5$ and $R^6$ are independently hydrogen, alkyl of 1 to 10 carbon atoms, cycloalkyl of 6 to 10 carbon atoms, aryl of 6 to 14 carbon atoms or halo groups,

provided that R′ is not hydrogen when R is alkyl as defined above, and further provided that when R and R′ are both either n-butyl, t-butyl or alkyl-substituted phenyl, at least one of $R^1$, $R^2$, $R^3$, $R^4$, $R^5$ and $R^6$ is alkyl, cycloalkyl, aryl or halo as defined above.

These compounds have various uses which are advantageous. In general, they are useful as ionophores in ion selective compositions, electrodes and methods, and particularly in lithium ion selective compositions, electrodes and methods.

The substituted 1,10-phenanthrolines of this invention are lipophilic, meaning that they are substantially insoluble in water. They are structurally defined in detail as follows:

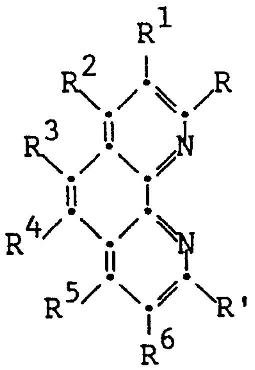

wherein R is selected from the group consisting of substituted or unsubstituted alkyl having a molecular weight of at least 45. Generally, this alkyl group has from 4 to 20 total carbon atoms in the group including any substituents (for example, n-butyl, t-butyl, n-pentyl, 2-chlorohexyl, decyl, dodecyl, n-butoxymethyl, benzyl, p-phenyloxymethyl and others known to one skilled in the art). However, smaller alkyl groups ( that is, having 1 to 3 carbon atoms) can be used as long as they have one or more substituents which provide a total molecular weight of at least 45. Such substituents include halo, alkoxy (such as methoxy, ethoxy, n-butoxy and others apparent to one skilled in the art), aryl (such as phenyl, naphthyl, xylyl, m-chlorophenyl and others known in the art), and other useful groups readily apparent to one skilled in the art. Compounds XI and XII illustrated below show representative examples of such groups.

R can also be substituted or unsubstituted cycloalkyl having 5 to 10 carbon atoms in the ring (for example, cyclopentyl, cyclohexyl, 4-methyl-cyclohexyl and others known to one skilled in the art) and substituted phenyl (especially alkyl-substituted phenyl wherein the alkyl being in any position on the phenyl ring, and being substituted or unsubstituted and having 1 to 10 carbon atoms, such as methyl, ethyl, isopropyl, n-butyl, hexyl, decyl, benzyl, methoxymethyl, chloromethyl and others known to one skilled in the art). Halo-substituted alkoxy-substituted and cycloalkyl-substituted phenyl are also useful with the substituents having the same definition as noted above.

In the structure above, R′ is either hydrogen, halo or, independently of R, selected from the groups defined for R.

$R^1$, $R^2$, $R^3$, $R^4$, $R^5$ and $R^6$ are independently of each other hydrogen, substituted or unsubstituted alkyl of 1 to 10 carbon atoms (for example methyl, chloromethyl, methoxymethyl, ethyl, propyl, isopropyl, octyl, decyl and others known to one skilled in the art), substituted or unsubstituted cycloalkyl of 6 to 10 carbon atoms as defined above for R, substituted or unsubstituted aryl of 6 to 10 carbon atoms (such as phenyl, p-methoxyphenyl, naphthyl, xylyl and others known in the art), or halo groups (for example fluoro, chloro, bromo or iodo).

However, in the definition above, R′ is not hydrogen when R is substituted or unsubstituted alkyl as defined above. Further, when R and R′ are both either n-butyl, t-butyl, or alkyl-substituted phenyl as defined above, at least one of $R^1$, $R^2$, $R^3$, $R^4$, $R^5$ and $R^6$ is alkyl, cycloalkyl, aryl or halo as defined above.

In the definition of R, R′ and $R^1$-$R^6$ herein, it is intended to include substituents which act as linkages to another 1,10-phenanthroline moiety, thereby providing a bis-compound. Compounds XII-XIV illustrated below are examples of such bis- embodiments of this invention.

In a preferred embodiment, in reference to the structure above, both R and R′ are either an unsubstituted linear alkyl of 4 to 12 carbon atoms (for example, n-butyl, n-hexyl, decyl, dodecyl or others known to one skilled in the art), or alkyl-substituted phenyl as defined above. R and R′ are identical in this embodiment. Also, $R^1$ and $R^6$ are both hydrogen, and $R^2$, $R^3$, $R^4$ and $R^5$ are independently of each other hydrogen, methyl or halo as defined above. However, in this preferred embodiment, when R and R′ are both n-butyl, at least one of $R^2$-$R^5$ is methyl or halo.

More preferably, R and R′ are the same linear alkyl of 4 to 8 carbon atoms, $R^1$ and $R^6$ are both hydrogen and $R^2$-$R^5$ are independently hydrogen or methyl.

Most preferably, R and R′ are the same linear alkyl of 4 to 6 carbon atoms, $R^1$ and $R^6$ are both hydrogen and $R^2$-$R^5$ are independently hydrogen or methyl provided either $R^2$ and $R^5$ are both methyl or $R^3$ and $R^4$ are both methyl.

Standard numbering of the positions of the phenanthroline structure identified above is used in the foregoing definition. Hence, the nitrogen atoms are in the 1- and 10-positions of the compound, R and R′ are in the 2- and 9-positions, respectively, and $R^1$-$R^6$ are in the 3- through 8-positions, respectively.

The preferred 1,10-phenanthrolines of this invention can also be defined in a functional manner using the following test. Generally, but not necessarily, the compounds exhibit a selectivity coefficient (k) of lithium ion over sodium ion of less than or equal to 0.1 as measured using a 0.1 molar solution of lithium chloride and 0.1 molar solution of sodium chloride. The selectivity coefficient is a well known parameter used in physical

chemistry and is measurable using well known procedures and equipment (see for example, Sugihara et al, Chem. Letters, 1987 pp. 2391-2392).

More preferably, the compounds of this invention exhibit a selectivity coefficient as defined above which is less than or equal to 0.01, and most preferably less than or equal to 0.0005.

The following list of substituted 1,10-phenanthrolines are representative of those provided by this invention:

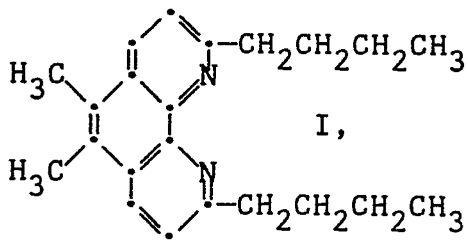
I,

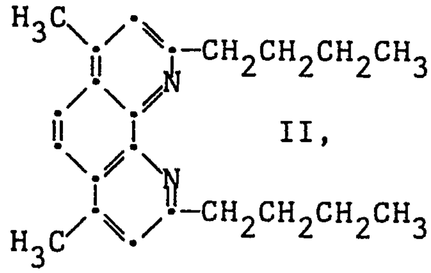
II,

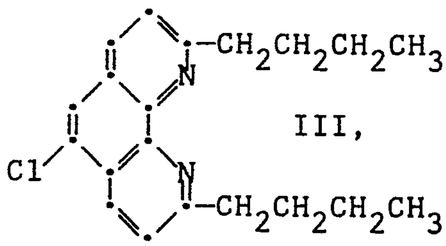
III,

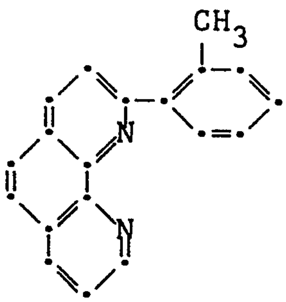
IV,

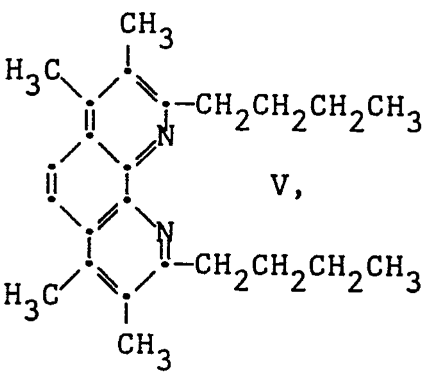
V,

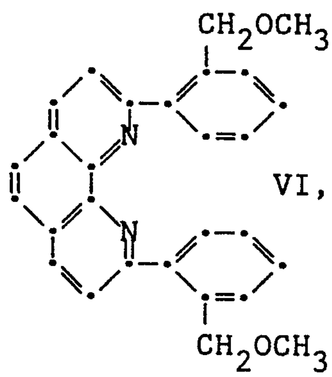
VI,

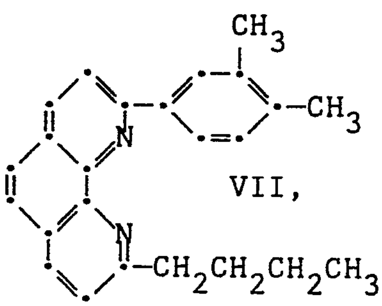
VII,

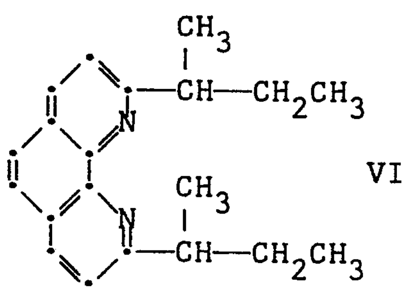
VIII,

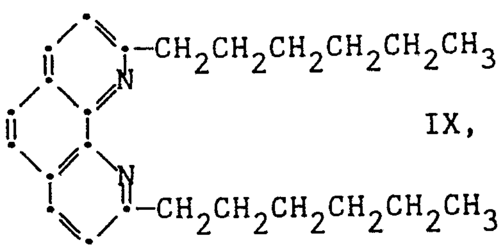

IX,

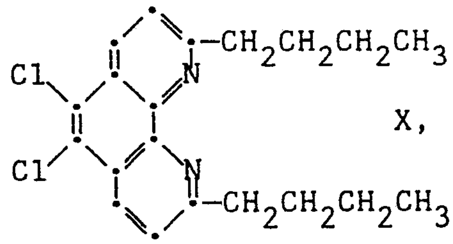

X,

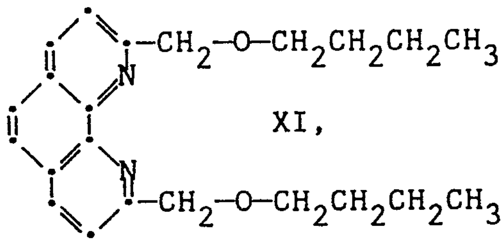

XI,

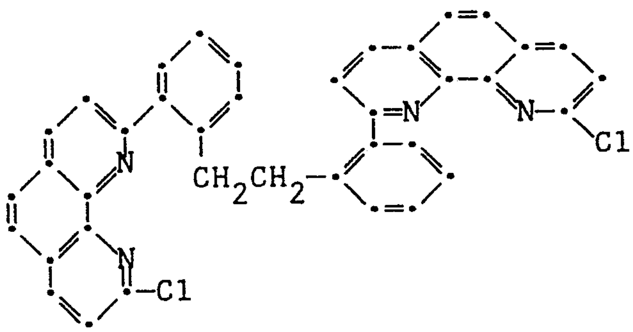

XII,

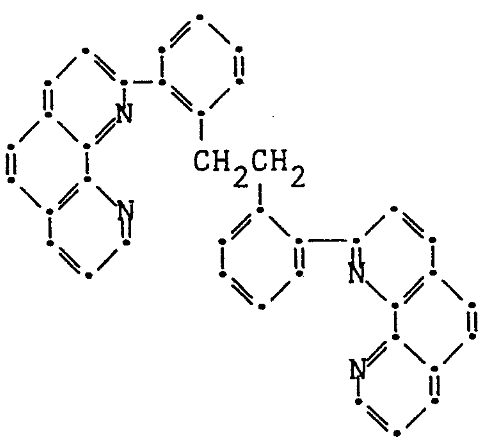

XIII,

The Compounds identified above as I, II, III, VI and IX are preferred for use as lithium ion-selective ionophores. Compounds I, II and IX are more preferred with Compounds I and II being most preferred.

In preparing the compounds of this invention, unsubstituted 1,10 phenanthrolines or 3- to 8-substituted-1,10-phenanthrolines are used as starting materials. Many of these reagents are commercially available. Others are readily prepared from known starting materials using known procedures.

The starting material is converted to the desired 2-, 9- or 2,9-disubstituted phenanthroline by first alkylating or arylating the dihydrolithium adduct at each of the positions desired to be substituted, generally by addition of an alkyl or aryl lithium reagent (prepared by known techniques) at a temperature between 0° and -70° C, quenching the excess lithium reagent with an alcohol (methanol) and oxidation of the two alkyl-hydro-substituted rings to the aromatic rings with removal of the proton using an oxidant such as free iodine.

Alternatively, the 2,9-dichloro or substituted 2,9-dichloro-1,10-phenanthroline starting materials can be converted to the 2-, 9- or 2,9-aryl derivatives by a Suzuki coupling reaction (Miyaura et al, Synthetic Commun., 11, pp. 513-519, 1981). The dichloro phenanthroline starting materials are coupled with an arylboronic acid, for example, a 2-methoxymethylphenylboronic acid when the desired substituent is a 2-methoxymethylphenyl

group, in the presence of tetrakis(triphenylphosphine)palladium catalyst. The methoxymethylphenylboronic acid is prepared by conversion of the corresponding aryl bromide to the corresponding aryl lithium with n-butyl lithium at low temperature (for example -78° C) and reaction with trimethylborate, again at low temperature. In this procedure, o-bromobenzylbromide is first refluxed with aqueous methanol in base to produce the 2-methoxymethylphenyl bromide which is converted in tetrahydrofuran at low temperature to the 2-methoxymethylphenyl lithium adduct before reaction with the trimethylborate.

Representative preparatory procedures are illustrated in the following examples. Starting materials are readily available from commercial sources.

Example 1: Preparation of 2,9-di-n-butyl-5,6-dimethyl-1,10-phenanthroline

5,6-Dimethyl-1,10-phenanthroline monohydrate (0.83 g, 4.2 mmolar) was dissolved in 25 ml of freshly distilled tetrahydrofuran in a flask equipped for magnetic stirring and having an argon inlet. The contents were cooled in an ice water bath and n-butyl lithium (10 ml, 2.1 molar solution in hexane) was added dropwise with a syringe through a septum attached to the flask. An immediate color change to dark black-purple and then to bright yellow occurred upon addition of each drop of n-butyl lithium until about 2 ml had been added. Thereafter, the solution remained a dark purple color. When all of the n-butyl lithium had been added, the cold bath was removed and the reaction mixture allowed to warm to room temperature overnight.

After recooling the reaction mixture, it was quenched by the addition of methanol (a few ml) and then oxidized by the addition of a solution of iodine (9 g in 30 ml tetrahydrofuran) for 30 minutes. After an additional 60 minutes of stirring, the crude product was isolated by pouring the reaction mixture into saturated sodium bisulfite solution and extracting with a mixture of ethyl ether and dichloro methane. The extracts were washed sequentially with bisulfite solution, water and brine, and then dried over anhydrous sodium sulfate. Removal of solvent under reduced pressure gave a dark oil which was purified by chromatography on silica gel using dichloromethane as eluant. In this manner, 0.7 g of the desired Compound I was obtained. The assigned structure was consistent with the proton NMR spectra.

Example 2: Preparation of 5-Chloro-2,9-Di-n-butyl-1,10-phenanthroline

5-Chloro-1,10-phenanthroline (5 mmole) was treated with n-butyl lithium (12.2 mmole) according to the procedure described in Example 1. The crude product (1.98 g) was purified by chromatography on 200 g of silica gel using methylene chloride as eluent to afford pure compound (435 mg) of Compound III as a light yellow solid. Field desorption mass spectoscopy gave a parent ion at m/e = 326. $^1$H NMR ($CDCl_3$) δ 0.97 (t,6H), 1.15-2.18 (m,8H), 3.05-3.4 (m,4H), 7.35 (d, J = 9, 1H), 7.47 (d, J = 9, 1H), 7.64 (s,1H), 7.9 (d, J = 9, 1H), 8.44 (d, J = 9, 1H).

Example 3: Preparation fo 5,6-Dichloro-2,9-di-n-butyl-1,10-phenanthroline

Compound X was prepared in a manner similar to Compound III in Example 1 above. The starting material was 5,6-dichloro-1,10-phenanthroline. Field desorption mass spectroscopy gave a parent ion at m/e = 360. $^1$H NMR ($CDCl_3$) δ 0.98 (t,6H), 1.28-2.1 (m,8H), 3.0-3.28 (m,4H), 7.56 (s,2H), 8.08 (s,2H).

Example 4 Preparation of 2,9-Di-(2-methoxymethylphenyl)-1,10-phenanthroline

Compound VI was prepared using the Suzuki coupling reaction (described by Miyaura et al, noted above) of 2,9-dichloro-1,10-phenanthroline (synthesized according to the procedures described by Halcrow et al, J.Chem.Soc., 1953, p. 2873 and Ogawa et al, J.Chem.Soc.,Chemical Commun., 1972, p. 577) and 2-methoxymethylphenylboronic acid.

The boronic acid was prepared as follows: Ortho-bromobenzyl bromide (0.25 mole) and methanol (50 ml) were stirred together in a round bottom flask at room temperature. To this mixture, potassium hydroxide (25 g) in methanol (150 ml) was added dropwise and the final mixture was heated to reflux for 2 hours. After cooling, the excess methanol was removed under reduced pressure and the residue extracted with ether. The ether extracts, after washing with water and brine solution, were dried over anhydrous sodium sulfate and the ether subsequently removed under reduced pressure. The product, 2-methoxymethylphenyl bromide, was purified by distillation (74% yield, b.p. 112° C/43 mm).

The corresponding boronic acid derivative was prepared in the following manner. A solution of the 2-methoxymethylphenyl bromide (0.04 mole) in freshly distilled tetrahydrofuran (100 ml) was cooled to -78° C in a dry ice/acetone bath. Conversion to the corresponding aryl lithium was effected by syringing n-butyl lithium (0.048 mole) into the reaction mixture. The solution was stirred for 10 minutes, during which time a precipitate of the aryl lithium began to form. The resulting suspension was transferred by cannula to a flask containing trimethylborate (0.10 mole) in dry tetrahydrofuran (100 ml) at -78° C. The reaction was allowed to warm to room temperature and added to a separatory funnel containing diethyl ether (400 ml) and 1N hydrochloric acid (100 ml). The organic layer was removed and the aqueous layer re-extracted with 100 ml of diethyl ether. The combined organic layers were extracted with 3N sodium hydroxide (3 times with 50 ml). The combined aqueous layers were neutralized with concentrated hydrochloric acid, and the organic products removed by extraction with diethyl ether. The combined ether layers were dried over anhydrous sodium sulfate and evaporated under vaccum to afford a very light yellow oil (3.98 g) which was used in the aryl coupling reaction without further purification.

A solution of 2,9-dichlorophenanthroline (3 mmole), toluene (20 ml), water (10 ml), sodium carbonate (2.12

g) and tetrakis(triphenylphosphine)palladium catalyst (35 mg) was prepared and purged with nitrogen. To this was added the boronic acid prepared above (6.6 mmole) followed by ethanol (5 ml). The resulting mixture was stirred vigorously and refluxed for 24 hours. An additional 35 mg of the catalyst was added and the refluxing continued for another 12 hours. The cooled reaction mixture was suction filtered through a Celite diatomaceous earth pad and then added to a separatory funnel containing water and dichloromethane. The organics were extracted with four separate portions of dichloromethane and the combined extracts were dried over anhydrous sodium sulfate and evaporated under vacuum. The crude product (1,4 g) was purified by chromatography on silica gel to afford 0.72 g (57% yield) of desired product. Field desorption mass spectroscopy gave a mass ion at m/e=420. $^1H$ NMR ($CDCl_3$) δ 3.32 (s,6H), 4.98 (s,4H), 7.2-8.3 (m,14H).

Example 5: Preparation of 2-n-butyl-9-(3,4-dimethylphenyl)-1,10-phenanthroline

A solution of 4-bromo-o-xylene (5.14 mmole) in freshly distilled tetrahydrofuran (50 ml) was cooled to -78°C in a nitrogen atmosphere. Tertiary-butyl lithium (10.3 mmole) was added by syringe and after 15 minutes at -78°C, the resulting lithio derivative was cannulated into a solution of 2-n-butyl-1,10-phenanthroline in freshly distilled tetrahydrofuran (50 ml) cooled to 0°C. After 15 minutes, the reaction was quenched by the addition of 5 ml of methanol and 4 drops of 1M sodium methoxide. A solution of iodine (4.6 g) in tetrahydrofuran was then added and the mixture stirred for one hour at room temperature. The product was isolated by extraction with a mixture of either and dichloromethane and purified by chromatography on silica gel to afford Compound VII as a colorless oil (18% yield). Field desorption mass spectoscopy gave a mass ion at m/e=340. $^1H$ NMR ($CDCl_3$) δ 0.98 (t,3H), 1.25-2.10 (m,4H), 2.25 (s,3H), 2.36 (s,3H), 3.2 (t,2H), 7.1-7.4 (m,9H).

Example 6: Preparation of 2,2′-Bis(9-chloro-1,10-phenanthrol-2-yl)bibenzyl

Compound XII was prepared using the Suzuki coupling reaction (as described in Example 4 above) of 2,9-dichloro-1,10-phenanthroline and the bis-boronic acid prepared from 2,2′-dibromobibenzyl. Field desorption mass spectroscopy gave mass ion at m/e=606. $^1H$ NMR ($CHCl_3$) δ 3.53 (s,4H), 7.0-8.35 (m,20H).

Example 7: Preparation of 2,9-Bis(butoxymethyl)-1,10-phenanthroline

The 2,9-bis(hydroxymethyl)-1,10-phenanthroline precursor to Compound XI was prepared according to the procedure of Chandler et al (J. Hetero.Chem., 18, 599, 1981). 2,9-Bis(hydroxymethyl)-1,10-phenanthroline (2 mmole) was treated with sodium hydride (5 mmole) in dry N,N-dimethylformamide (10 ml). N-butyl iodide (5 mmole) was added and the reaction mixture was stirred for six hours at room temperature. The reaction mixture was then poured into 50 ml of 5% aqueous sodium bicarbonate and extracted with ethyl acetate three times. The organic extracts were combined and dried over anhydrous sodium sulfate. The solvent was removed under reduced pressure, and the crude product was chromatographed on silica gel using 10% ethyl acetate and 2% isopropanol in methylene chloride as eluant to yield 150 mg (20.0%) of a yellow solid. NMR ($CDCl_3$) δ 0.95 (t,6H), 1.25-1.85 (m,8H), 3.67 (t,4H), 5.1 (s,4H), 7.75 (s,2H), 7.87 (d,2H), 8.3 (d,2H).

Example 8: Preparation of 2,9-di-sec-butyl-1,10-phenanthroline

Compound VIII was prepared by dissolving phenanthroline monohydrate (0.83 g, 4.2 mmole) in freshly distilled tetrahydrofuran (25 ml) in a 50 ml flask equipped for stirring and having an argon inlet. The contents were cooled in an ice water bath and sec-butyl lithium (10 ml, 2.1 molar solution in hexane) was added dropwise using a syringe through a septum on the flask. An immediate color change to dark, purple-black and then to bright yellow occurred upon addition of each drop of sec-butyl lithium until about 2 ml were added. Thereafter, the solution maintained a dark purple color. After the addition of sec-butyl lithium was complete, the cold bath was removed and the reaction mixture allowed to warm to room temperature overnight.

After recooling the reaction mixture, it was quenched by the addition of a few ml of methanol and then oxidized by the addition of a solution of iodine (9 g) in tetrahydrofuran (30 ml) for 30 minutes. After an additional hour of stirring, the crude product was isolated by pouring the reaction mixture into saturated sodium bisulfite solution and extracting with a mixture of diethyl ether and dichloromethane. The extracts were washed with bisulfite solution, water, brine, and then dried over anhydrous sodium sulfate. Removal of solvent under reduced pressure afforded a dark oil which was purified by chromatography on silica gel using dichloromethane as eluant. In this manner, Compound VIII (0.7 g) was obtained.

Example 9: Preparation of 2,9-Di-n-butyl-4,7-dimethyl-1,10-Phenanthroline

Compound II was prepared by treating 4,7-dimethyl-1,10-phenanthroline (5 mmole) with n-butyl lithium (20 mmole) according to the procedure described in Example 1. The resulting crude Compound II was purified by silica gel chromatography using 15% acetonitrile in toluene as eluant to yield 1.1 g (68.8%) of a pale yellow solid. Proton NMR ($CDCl_3$): δ 0.98 (t,6H), 1.28-2.10 (m,8H), 2.7 (s,6H), 3.0-3.28 (m,4H), 7.4 (s,2H), 7.93 (s,2H).

Example 10: Preparation of 2-(o-tolyl)-1,10-Phenanthroline

o-Bromotoluene (52 mmole) was dissolved in freshly distilled tetrahydrofuran (40 ml) under an argon atmosphere. This solution was then cooled to -78°C and sec-butyl lithium (57 mmole) was added dropwise, stirred an additional 40 minutes and then warmed to 0°C. Phenanthroline monohydrate (20 mmole) was then treated with the o-tolyl lithium in a manner similar to that employed in the preparation of Compound VII in Example 5, except that the methanol quench was done five minutes after the completion of the addition of

o-tolyl lithium.

The resulting crude Compound IV was chromatographed on silica gel using 25% acetonitrile in toluene as eluant yielding 3.5 g (70.0%) of a yellow solid. Proton NMR ($CDCl_3$): δ 2.45 (s,3h), 7.24-7.40 (m,3H),7.5-7.75 (m,3H), 7.62 (s,2H), 8.15-8.35 (m,2H), 9.2-9.35 (m,1H).

Example 11: Preparation of 3,4,7,8-Tetramethyl-2,9-di-n-butyl-1,10-Phenanthroline

Compound V was prepared by treating 3,4,7,8-tetramethyl-2-n-butyl-1,10-phenanthroline (0.9 mmole) with n-butyl lithium (4.5) in a manner similar to Example 1. The crude product was purified by silica gel chromatography using 25% acetonitrile in toluene as eluant to yield 40 mg (12.7%) of a pale yellow solid. Proton NMR ($CDCl_3$): δ 0.98 (t,6H), 1.28-2.10 (m,8H), 2.45 (s,6H), 2.6(s,6H), 3.0-3.28 (m,4H), 7.95 (s,2H).

Example 12: Preparation of 2,9-di-n-Hexyl-1,10-Phenanthroline

n-Hexyl lithium was prepared by the method of Corey, Tetra. Lett., 44, 3847, 1977, and 2-n-hexyl-1,10-phenanthroline (1.7 mmole) was treated with the n-hexyl lithium (3.4 mmole) in a manner similar to Example 1, except that the methanol quench was done one hour after the completion of the lithium treatment. The crude Compound IX was purified by silica gel chromatography using 3% acetonitrile in toluene as eluant yielding a light yellow oil. Proton NMR ($CDCl_3$): δ 0.98 (t,6H), 1.28-2.10 (m,16H), 3.0-3.28 (m,4H), 7.5 (d,2H), 7.7 (s,2H), 8.15 (d,2H).

Use of Substituted Phenanthrolines:

Several substituted 1,10-phenanthrolines of this invention were used in lithium ion-selective electrodes. The following Table I lists the tested compounds and their coefficients.

TABLE I

| Compound | $k(Li^+/Na^+)$ |
|---|---|
| I | 0.004 |
| II | 0.005 |
| III | 0.025 |
| IV | 0.14 |
| V | 0.90 |
| VI | 0.058 |
| VII | 0.019 |
| VIII | 0.0073 |
| IX | 0.009 |
| X | 0.011 |
| XI | 0.98 |

**Claims**

1. A substituted 1,10-phenanthroline characterized as having the structure:

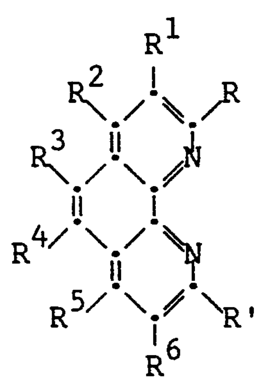

whereinR is selected from the group consisting of alkyl having a molecular weight of at least 45, cycloalkyl having 5 to 14 carbon atoms in the ring and substituted phenyl,

R′ is either hydrogen, halo or independently selected from the groups defined for R,

$R^1$, $R^2$, $R^3$, $R^4$, $R^5$ and $R^6$ are independently hydrogen, alkyl of 1 to 10 carbon atoms, cycloalkyl of 6 to 10

carbon atoms, aryl of 6 to 10 carbon atoms or halo groups,
provided that R′ is not hydrogen when R is alkyl as defined above, and further provided that when R and R′ are both either n-butyl, t-butyl or alkyl-substituted phenyl, at least one of $R^1$, $R^2$, $R^3$, $R^4$, $R^5$ and $R^6$ is alkyl, cycloalkyl, aryl or halo as defined above.

2. The 1,10-phenanthroline as claimed in claim 1 which exhibits a selectively coefficient of lithium ion over sodium ion of less than or equal to 0.1 as measured using a 0.1 molar solution of lithium chloride and 0.1 molar solution of sodium chloride.

3. The 1,10-phenanthroline as claimed in either of claims 1 or 2 wherein both R and R′ are either a linear alkyl of 4 to 12 carbon atoms or alkyl-substituted phenyl, $R^1$ and $R^6$ are hydrogen, and $R^2$-$R^5$ are independently hydrogen, methyl or halo,
provided that when R and R′ both are n-butyl or t-butyl, at least one of $R^2$-$R^5$ is methyl or halo, and further provided that said compound exhibits a selectivity coefficient of lithium ion over sodium ion of less than or equal to 0.1 as measured using a 0.1 molar solution of lithium chloride and 0.1 molar solution of sodium chloride.

4. The 1,10-phenanthroline as claimed in any of claims 1 to 3 wherein R and R′ are both linear alkyl of 4 to 8 carbon atoms, $R^1$ and $R^6$ are hydrogen, and $R^2$-$R^5$ are independently hydrogen or methyl, provided that said compound exhibits a selectivity coefficient of lithium ion over sodium ion of less than or equal to 0.01 as measured using a 0.1 molar solution of lithium chloride and 0.1 molar solution of sodium chloride.

5. The 1,10-phenanthroline as claimed in any of claims 1 to 4 wherein R and R′ both are linear alkyl of 4 to 6 carbon atoms, $R^1$ and $R^6$ are hydrogen, and $R^2$-$R^5$ are independently hydrogen or methyl provided either $R^2$ and $R^5$ are both methyl or $R^3$ and $R^4$ are both methyl, provided that said compound exhibits a selectivity coefficient of lithium ion over sodium ion of less than or equal to 0.005 as measured using a 0.1 molar solution of sodium lithium chloride and 0.1 molar solution of sodium chloride.

6. The 1,10-phenanthroline as claimed in any of claims 1 to 5 which is any of the following:

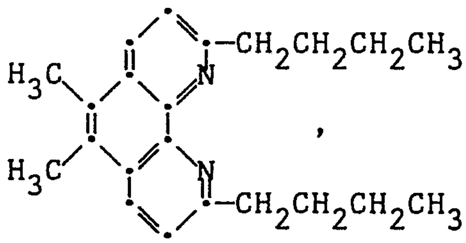

,

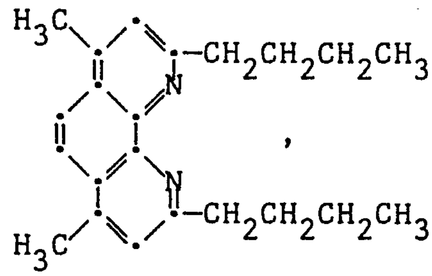

,

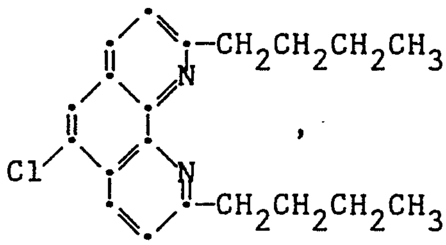

,

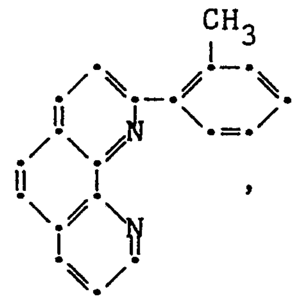

,

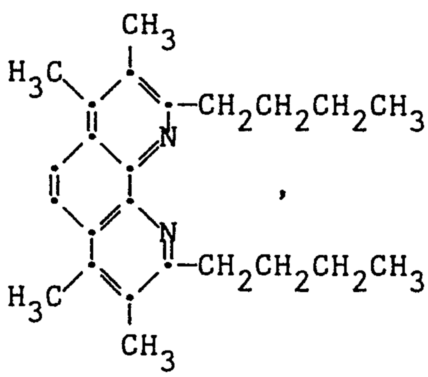

,

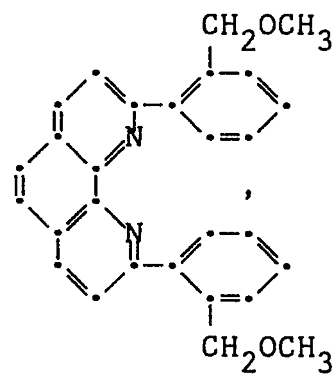

,

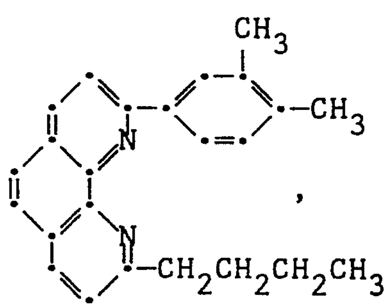

,

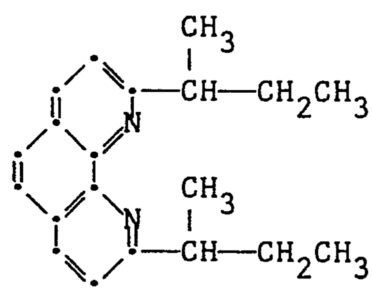

,

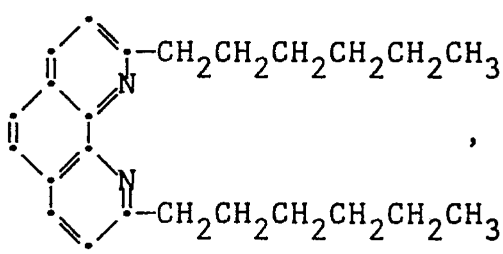

,

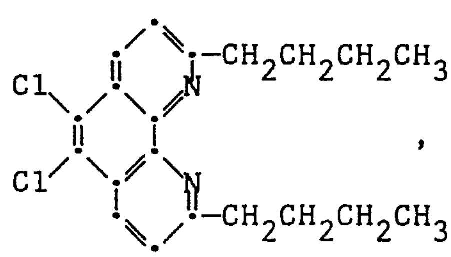

,

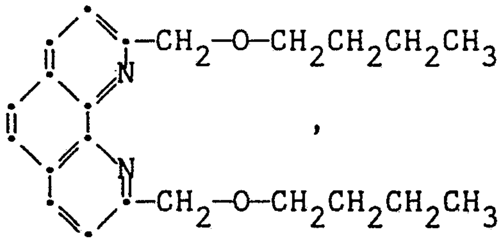

,

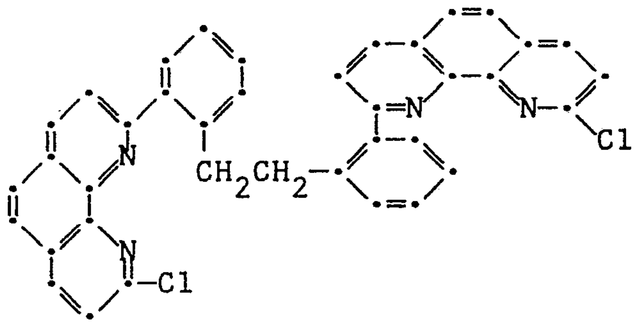

,

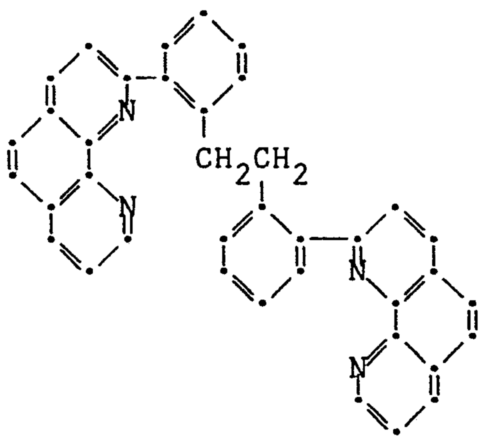

,

7. The substituted 1,10-phenanthroline

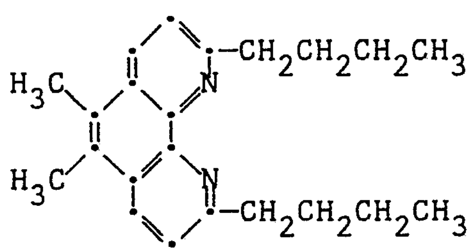

.

8. The substituted 1,10-phenanthroline

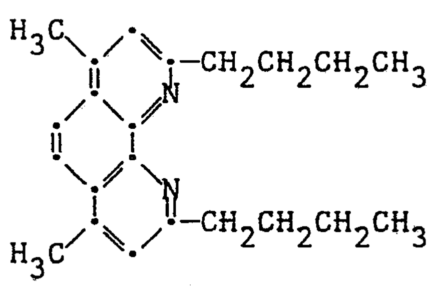

.

European Patent Office

# EUROPEAN SEARCH REPORT

Application Number

EP 89 30 4163

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | TETRAHEDRON LETTERS, vol. 28, no. 47, 1987, pages 5829-5832, Pergamon Journals Ltd, Oxford, GB; S. NOBLAT et al.: "Synthesis of an oblique bis-porphyrin system containing A 1,10-phenanthroline spacer" * Page 5831, compounds 5,6 * --- | 1 | C 07 D 471/04<br>C 07 D 519/00 //<br>(C 07 D 471/04<br>C 07 D 221:00<br>C 07 D 221:00 )<br>(C 07 D 519/00<br>C 07 D 471:00<br>C 07 D 471:00 ) |
| X | JOURNAL OF THE CHEMICAL SOCIETY, CHEMICAL COMMUNICATIONS, 1986, pages 1376-1378, London, GB; C.O. DIETRICH-BUCHECKER et al.: "High-yield synthesis of multiring copper(1) catenates by acetylenic oxidative coupling" * Page 1377, compounds 1,2 * --- | 1 | |
| X | TETRAHEDRON LETTERS, vol. 27, no. 20, 1986, pages 2257-2260, Pergamon Journals Ltd, Oxford, GB; C.O. DIETRICH-BUCHECKER et al.: "Interlocked macrocyclic ligands: A catenand whose rotation of one ring into the other is precluded by bulky substituents" * Page 2258, compounds 1,2 * --- | 11 | **TECHNICAL FIELDS SEARCHED (Int. Cl.4)**<br>C 07 D 471/00<br>C 07 D 519/00 |
| X | EP-A-0 171 978 (HSC) * Page 20, example 2a * --- -/- | 11 | |

The present search report has been drawn up for all claims

| Place of search | Date of completion of the search | Examiner |
|---|---|---|
| THE HAGUE | 10-07-1989 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 89 30 4163

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | JOURNAL OF THE AMERICAN CHEMICAL SOCIETY, vol. 110, 2nd march 1988, pages 1467-1472, American Chemical Society, Washington, US; A.-M. ALBRECHT-GARY et al.: "Topological kinetic effects: Complexation of interlocked macrocyclic ligands by cationic species" * Page 1468, formula "dap" * --- | 1 | |
| X | US-A-4 598 073 (RESEARCH CORP.) * Example 21: "Starting product" * --- | 1 | |
| X | CHEMICAL ABSTRACTS, vol. 96, 1982, page 647, abstract no. 35042z, Columbus, Ohio, US; N.S. KOZLOV et al.: "Synthesis of aminals from 8-aminoquinoline", & KHIM. GETEROTSIKL. SOEDIN. 1981, (10), 1379-81 * Formula IV * --- | 1 | TECHNICAL FIELDS SEARCHED (Int. Cl.4) |
| X | JOURNAL OF HETEROCYCLIC CHEMISTRY, vol. 18, May 1981, pages 599-601, Hetero Corp., Tampa, FL, US; C.J. CHANDLER et al.: "Synthesis of some 2,9-disubstituted-1,10-phenanthrolines" * Compounds 10,11,12 * --- | 1 | |
| X | CHEMICAL ABSTRACTS, vol. 80, 1974, page 412, abstract no. 95912n, Columbus, Ohio, US; & CS-A-150 748 (B. ZAK) 15-10-1973 * Abstract * --- -/- | 1 | |

The present search report has been drawn up for all claims

| Place of search | Date of completion of the search | Examiner |
|---|---|---|
| THE HAGUE | 10-07-1989 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 89 30 4163

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | CHEMICAL ABSTRACTS, vol. 108, 1988, page 716, abstract no. 48291w, Columbus, Ohio, US; H. SUGIHARA et al.: "Lithium ion-selective electrodes based on 1,10-phenanthroline derivatives", & CHEM. LETT. 1987, (12), 2391-2 * Abstract * --- | 1 | |
| P,X | CHEMICAL ABSTRACTS, vol. 109, 1988, page 690, abstract no. 128980f, Columbus, Ohio, US; D.K. MITCHELL et al.: "A topologically chiral [2]catenand", & ANGEW. CHEM. 1988, 100(7), 985-7 * Compound L * --- | 1 | |
| P,X | EP-A-0 282 893 (BAYER) * Page 25, example 6; page 33, example (I)-104 * --- | 1 | |
| P,X | JOURNAL OF ORGANIC CHEMISTRY, vol. 54, 1989, pages 1766-1769, American Chemical Society, Washington, US; G.R. NEWKOME et al.: "Mono-alpha-functionalization of 2,9-dimethyl-1,10-phenanthroline" * Page 1767, compounds 6a,6b * ----- | 1 | TECHNICAL FIELDS SEARCHED (Int. Cl.4) |

The present search report has been drawn up for all claims

| Place of search | Date of completion of the search | Examiner |
|---|---|---|
| THE HAGUE | 10-07-1989 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document